(19) Europäisches Patentamt
European Patent Office
Office européen des brevets

(11) Veröffentlichungsnummer: **0 126 886**
**B1**

(12) # EUROPÄISCHE PATENTSCHRIFT

(45) Veröffentlichungstag der Patentschrift: **11.06.86**

(21) Anmeldenummer: **84103329.3**

(22) Anmeldetag: **27.03.84**

(51) Int. Cl.⁴: **C 07 C 99/12**, C 07 C 101/04, C 12 P 13/06

(54) **Verfahren zur Trennung von L-Leucin und L-Isoleucin.**

(30) Priorität: **25.05.83 DE 3318933**

(43) Veröffentlichungstag der Anmeldung: **05.12.84 Patentblatt 84/49**

(45) Bekanntmachung des Hinweises auf die Patenterteilung: **11.06.86 Patentblatt 86/24**

(84) Benannte Vertragsstaaten: **AT BE CH FR GB IT LI NL SE**

(56) Entgegenhaltungen:
**CH - A - 284 412**

**LIEBIGS ANNALEN DER CHEMIE, Heft 11, 15 November 1983, Seiten 2052-2054, Verlag Chemie GmbH, WEINHEIM, (DE), J. MARTENS et al.: "Enzymatic separation of 1-leucine and 1-isoleucine"**

(73) Patentinhaber: **Degussa Aktiengesellschaft, Weissfrauenstrasse 9, D-6000 Frankfurt am Main 1 (DE)**

(72) Erfinder: **Kleemann, Axel, Dr. Dipl.-Chem., Greifenhagenstrasse 25, D-6450 Hanau 9 (DE)**
Erfinder: **Martens, Jürgen, Dr. Dipl.-Chem., Hochstrasse 10, D-8755 Alzenau (DE)**
Erfinder: **Weigel, Horst, Odenwaldstrasse 56, D-6458 Rodenbach (DE)**

EP 0 126 886 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft ein Verfahren zur Trennung von L-Leucin und L-Isoleucin in Aminosäuregemischen, die 30 bis 65 Gewichtsprozent L-Leucin, 35 bis 70 Gewichtsprozent L-Isoleucin und höchstens 35 Gewichtsprozent an anderen Aminosäuren, jeweils bezogen auf Trockensubstanz, enthalten.

Die Aminosäuren Leucin und Isoleucin haben die gleiche Summenformel $C_6H_{13}NO_2$ und unterscheiden sich nur durch die Struktur der verzweigten aliphatischen Seitenkette R:

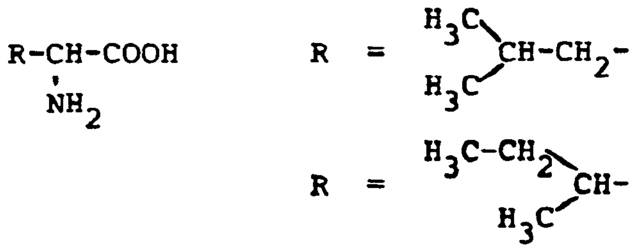

Leucin

Isoleucin

Bedingt durch diese sehr ähnlichen Strukturmerkmale zeigen diese Aminosäuren im physikalischen und chemischen Verhalten sehr ähnliche Eigenschaften, und die Trennung bereitet große Schwierigkeiten.

Die erste Trennung von Leucin und Isoleucin wurde über die Kupferkomplexe durchgeführt. Dabei werden die getrockneten Kupferkomplexe der Aminosäuren mit Methanol extrahiert, das Kupfersalz von Isoleucin geht hierbei in Lösung. In ähnlichen Verfahren werden auch die Kobaltkomplexe der Aminosäuren durch Extraktion mit Alkohol aufgetrennt. Bei diesen Verfahren ergibt sich jedoch das Problem der Rückgewinnung der Metalle und der weiteren Reinigung der Aminosäuren.

Von anderen Autoren wurde die Trennung von Leucin und Isoleucin mit aromatischen Sulfonsäuren beschrieben. So wurde die Verwendung von 2-Bromtoluol-5-sulfonsäure oder Naptha-lin-2-sulfonsäure für die Fällung von Leucin und von l-Chlor-4-naphthalinsulfonsäure oder 2-Naphthol-6-sulfonsäure für die Fällung von Isoleucin vorgeschlagen. Auch Benzolsulfonsäure sowie p-Toluolsulfonsäure wurden für die Leucin/ Isolucin-Trennung eingesetzt. Bei diesen Verfahren müssen die Präzipitate durch zahlreiche Umkristallisationen gereinigt werden und ein besonderes Problem bedeutet die Abtrennung der oft stark giftigen Fällungsmittel.

In einem weiteren bekannten Verfahren werden Gemische aus Isoleucin, Leucin und Valin durch vielstufige Kristallisation von L-Leucin im pH-Bereich 1,5 bis 2,0 getrennt und anschließend L-Isoleucin-Hydrochlorid durch Kristallisation aus konzentrierter Salzsäure gewonnen, wobei sich das restliche Leucin in der Mutterlauge anreichert. Nach diesem Verfahren läßt sich L-Leucin jedoch nicht in für pharmazeutische Anwendungen ausreichender Reinheit gewinnen.

Das erfindungsgemäße Verfahren ist nun dadurch gekennzeichnet, daß man

a) das Aminosäuregemisch in an sich bekannter Weise acetyliert,

b) aus dem rohen Gemisch der Acetylierungsprodukte durch Ansäuern mit einer Mineralsäure ein an N-Acetyl-L-leucin und N-Acetyl-L-isoleucin angereichertes Gemisch an N-Acetylaminosäuren ausfällt,

c) dieses angereicherte Gemisch in wässriger Lösung mit einer Konzentration zwischen 0,1 und 1,5 Mol/l an N-Acetylaminosäuren insgesamt bei einem pH zwischen 6 und 8 und einer Temperatur zwischen 10 und 40°C in Gegenwart eines Effektors einer Verseifung durch eine L-Aminosäureacylase unterwirft, bis 25 bis 95 % des eingesetzten N-Acetyl-L-leucins zur freien Aminosäure verseift sind,

d) aus dem rohen Verseifungsgemisch reines L-Leucin auskristallisiert und abtrennt,

e) aus der nach dem Abtrennen des L-Leucins verbleibenden Mutterlauge N-Acetyl-L-isoleucin isoliert und

f) das N-Acetyl-L-isoleucin in an sich bekannter Weise zur freien Aminosäure verseift.

Als Ausgangsmaterialien für das erfindungsgemäße Verfahren können Aminosäuregemische dienen, die nur aus Leucin und Isoleucin bestehen. Ebenso können aber auch Aminosäuregemische eingesetzt werden, die außerdem noch andere Aminosäuren enthalten. Solche Aminosäuregemische fallen bei der Verarbeitung von Proteinguellen, wie entzuckerten Melassen, Getreide- oder Maiskeimen, Oeltrester, Mikroorganismen, insbesondere Hefen, oder Kasein, sowie Keratinen (Haare, Borsten, Federn, Hornspäne) und Leder an.

Bei der Hydrolyse solcher Proteinquellen fallen oft schwerlösliche Rückstände an, die reich an Leucin und Isoleucin sind. Ebenso erhält man aus den Hydrolysaten durch chromatographische Verfahren (Ionenaustausch, Ionenausschluß und/ oder Molekularsiebeffekt) Fraktionen die reich an Leucin und Isoleucin sind. Solche Fraktionen können außerdem noch Verunreinigungen, insbesondere anorganische Salze, wie Natriumchlorid, Natriumsulfat, Ammoniumchlorid oder Ammoniumsulfat enthalten.

Für das erfindungsgemäße Verfahren besonders geeignet sind Aminosäuregemische, die 40 bis 65 Gewichtsprozent Leucin, 35 bis 60 Gewichtsprozent Isoleucin und maximal 20 Gewichtsprozent andere Aminosäuren enthalten.

Das Aminosäuregemisch wird zunächst in an sich bekannter Weise acetyliert. Die Acetylierung kann mit Acetylchlorid oder Acetanhydrid oder auch mit Keten nach dem aus der DE-OS 27 41 081 bekannten Verfahren vorgenommen werden.

Anschließend wird aus dem rohen Gemisch der Acetylierungsprodukte durch Ansäuern mit einer Mineralsäure, z.B. Salzsäure oder Schwefelsäure, ein Gemisch von N-Acetylaminosäuren ausgefällt. Zweckmäßigerweise wird dabei auf einen pH zwischen 0,5 und 2 angesäuert. Dabei findet bereits eine Anreicherung von N-Acetyl-L-leucin und N-Acetyl-Lisoleucin statt. Der etwaige Gehalt an anderen N-Acetylaminosäuren als N-Acetyl-L-leucin und N-Acetyl-L-isoleucin nimmt ab. Durch eine anschließende Umkristallisation aus Wasser, einem mit Wasser mischbaren aliphatischen Alkohol mit 1 bis 4 Kohlenstoffatomen, wie Methanol, Ethanol, n-Propanol, Isopropylalkohol, n-Butanol, Isobutylalkohol oder tert. Butylalkohol oder einem Gemisch von Wasser und einem solchen Alkohol kann der lehalt an sonstigen N-Acetylaminosäuren auf normalerweise weniger als 4, in vielen Fällen weniger als 1 Gewichtsprozent. bezogen auf Trockensubstanz, vermindert werden.

Das an N-Acetyl-L-leucin und N-Acetyl-L-isoleucin angereicherte Gemisch wird nun der Verseifung durch eine L-Aminosäureacylase unterworfen. Die Einstellung des pH auf den Bereich zwischen 6 und 8 kann beispielsweise durch Ammoniak, vorzugsweise aber durch Natronlauge erfolgen. Als Effektoren können die üblicherweise bei der Racematspaltung von N-Acetyl-DL-k-aminocarbonsäuren mittels einer L-Aminosäureacylase zugesetzten dienen, beispielsweise die Ionen $Ca^{2+}$, $Fe^{2+}$, $Mn^{2+}$, Mg, Zn und vorzugsweise $Co^{2+}$. Sie werden zweckmäßigerweise in einer Konzentration zwischen $1. 10^{-5}$ und $1. 10^{-1}$ Mol/l und beispielsweise in Form der entsprechenden Chloride zu esetzt In vielen Fällen ist es vorteilhaft, dem Reaktionsgemisch zusätzlich geringer Mengen eines Biocids, beispielsweise p-Hydroxybenzoesäure-npropylester, zuzusetzen.

Als L-Aminosäureacylase wird bevorzugt eine Nierenacylase eingesetzt. Sie kann entweder in der handelsüblichen nativen Form angewandt werden oder als Immobilisat auf organischen Trägermaterialien, wie vernetzter Agarose, Dextrangelen, Cellulose, Hydroxyethylcellulose oder Mischpolymerisaten von Acrylamid mit vernetzenden Comonomeren, oder auf anorganischen Trägermaterialien, wie verschiedenen porösen oxidischen Materialien oder insbesondere Glaskügelchen.

Bei Anwendung einer relativ großen Menge an L-Aminosäureacylase kann die für die Verseifung erforderliche Reaktionszeit verkürzt werden. Nimmt man längere Reaktionszeiten in Kauf, kann die Einsatzmenge an L-Aminosaureacylase erheblich vermindert werden. Zwischen der Reaktionszeit und der Einsatzmenge an L-Aminosäureacylase besteht demnach eine ausgeprägte gegenseitige Abhängigkeit.

Die Verseifung, also die Reaktionsstufe c), wird dann abgebrochen, wenn 25 bis 95 %, vorzugsweise 30 bis 85 %, insbesondere 50 bis 80 %, des ursprünglich vorhandenen N-Acetyl-L-leucins zum freien L-Leucin verseift sind. Die Verseifungsreaktion wird analytisch verfolgt, z.B. mittels Hochdruckflüssigkeitschromatographie oder mit einem Aminosäureanalysator.

Die Verseifungsreaktion kann diskontinuierlich oder kontinuierlich durchgeführt werden. Für die diskontinuierliche Reaktionsführung arbeitet man in einem Rührkessel oder in einem Tank mit Umlauf. Eine kontinuierliche Reaktionsführung kann beispielsweise mittels eines Enzym-Membranreaktors realisiert werden.

Weist das für die Verseifungsreaktion eingesetzte Gemisch eine relativ hohe Konzentration an N-Acetyl-L-leucin auf, beispielsweise zwischen 1,0 und 1,5 Mol/l, so beginnt schon nach kurzer Zeit ein farbloser Niederschlag auszukristallisieren, der aus reinem L-Leucin besteht. Wird das Gemisch in niedrigeren Konzentrationen eingesetzt, ist es zweckmäßig, das rohe Verseifungsgemisch unter schonenden Bedingungen aufzukonzentrieren, wobei dann ebenfalls reines L-Leucin auskristallisiert. In beiden Fällen wird das reine L-Leucin durch Abfiltrieren oder Abzentrifugieren isoliert, gegebenenfalls nach vorherigem Klassifizieren zwecks Abtrennung einer etwa eingesetzten immobilisierten L-Aminosäureacylase.

Wird die Verseifung mit nativer L-Aminosäureacylase vorgenommen, so kann diese nach der Abtrennung des L-Leucins durch Ultrafiltration, z.B. an Hohlfasermembranen, zurückgewonnen und erneut eingesetzt werden.

Unter Umständen kann es vorteilhaft sein, das L-Leucin in mehreren Fraktionen zu isolieren, d.h. nach Abtrennung einer ersten Fraktion die Verseifung fortzusetzen und weitere Fraktionen von L-Leucin zu gewinnen. Eine gegebenenfalls wünschenswerte weitere Reinigung der einzelnen Fraktionen oder der Gesamtmenge an abgetrenntem L-Leucin kann durch Umkristallisation aus Wasser erfolgen.

In der nach Abtrennung des L-Lercins und gegebenenfalls der L-Aminosäureacylase verbleibenden Mutterlauge ist das N-Acetyl-L-isoleucin merklich angereichert. Dieses kann auf an sich bekannte Weise durch Extraktion, beispielsweise mit Ethylacetat, fraktionierte Kristallisation oder mittels eines Ionenaustauschers isoliert werden. Die in der Mutterlauge normalerweise ebenfalls noch enthaltenen freien Aminosäuren L-Leucin und L-Isoleucin können zurückgewonnen und wieder in die Acetylierungsstufe zurückgeführt werden.

Schließlich wird das N-Acetyl-L-isoleucin in an sich bekannter Weise, z.B. mit Salzsäure, zur freien Aminosäure verseift. Diese kann durch Kristallisation als Hydrochlorid weiter gereinigt werden. Das gereinigte Hydrochlorid wird dann mittels eines schwach basischen Ionenaustauschers oder durch Auflösen in Ethanol und Neutralisieren mit einem tertiären Amin, beispielsweise Triethylamin, in reines L-Isoleucin überführt.

Das erfindungsgemäße Verfahren hat gegenüber den bekannten Verfahren zur Trennung von Leucin und Isoleucin einen weiteren wesentlichen Vorteil. Bei der im industriellen Maßstab betriebenen Hydrolyse von Proteinen tritt eine teilweise Racemisierung des L-Leucins ein. Wenn man das L-Leucin nun nach dem erfindungsgemäßen Verfahren gewinnt, so ist das gewonnene Produkt von hoher optischer Reinheit, da die Acylase enantiospezifisch nur die Hydrolyse von N-Acetyl-L-aminosäuren katalysiert, während die N-Acetyl-D-aminosäuren unverändert zurückbleiben.

Die Erfindung wird durch die nachfolgenden Beispiele näher erläutert. Für diese Beispiele gilt folgendes:

Die Aktivität der L-Aminosäureacylase wird in units (U) angegeben. 1 U verseift bei pH 7,0 und 25°C 1 µMol N-Acetyl-L-methionin pro Stunde.

Die für das L-Leucin und das L-Isoleucin angegebenen Drehwerte $[\alpha]_D^{25}$ werden bei c = 4 in 6N Salzsäure gemessen. Die Drehwerte der reinen Aminosäuren in Pharmaqualität betragen laut US-Pharmacopoe XX unter diesen Bedingungen

L-Leucin $[\alpha]^2{}_D{}^5$ = +14,9° bis +17,3°

L-Isoleucin$[\alpha]_D^{25}$ = +38,9° bis +41 o°

Die Bestimmung der Zusammensetzung der eingesetzten Aminosäuregemische erfolgt mit einem Aminosäureanalysator.

Die Bestimmung der Zusammensetzung der zur enzymatischen Verseifung eingesetzten Gemische von N-Acetylaminosäuren erfolgt über die Drehwerte.

Prozentangaben bedeuten, sofern nicht anders angegeben, Gewichtsprozente.

**Beispiel 1:**

Eine als braunes Pulver erhaltene Fraktion neutraler Aminosäuren aus der chromatographischen Trennung eines Keratinhydrolysates hat folgende Zusammensetzung an Aminosäuren:

Leu 49,8%
Ile 36,1 %
Val 3,5 %
Met 3,5 %
Tyr 2,5 %
Ala 2,1 %
Phe 1,6 %
Gly 0,9 %

525 g dieser Mischung wurden in 2 Litern 2N Natronlauge gelöst und auf 0°C gekühlt. Innerhalb 1 Stunde wurden 410 ml Acetanhydrid und gleichzeitig 850 ml 5N Natronlauge eingerührt, wobei darauf geachtet wurde, daß der pH-Wert in einem Bereich zwischen pH 10 und 12 blieb und die Temperatur +5°C nicht überschritt. Nach beendeter Zugabe wurde noch 1 Stunde lang bei 5°C gerührt.

Die braune Lösung wurde mit konzentrierter Salzsäure verrührt, bis pH 1,5 erreicht war.

Durch Absaugen, Waschen mit Wasser und Trocknen, ergaben sich 530 g Acetylverbindungen als schwach braun gefärbtes Pulver. Dieses Rohprodukt wurde aus n-Butanol umkristallisiert, wobei 335 g einer farblosen Mischung aus 65 % N-Acetyl-leucin und 35 % N-Acetyl-isoleucin erhalten wurden.

86,6 g dieser Mischung aus N-Acetyl-leucin und N-Acetylisoleucin wurden in 500 ml IN Natronlauge gelöst. Durch Zugabe von wenigen Tropfen Lauge wurde der pH-Wert auf 7,5 gestellt.

Als Katalysator wurden 500 mg Acylase aus Schweinenieren (1200 U/mg) und 60 mg $CoCl_2 \times 6H_2O$ zugegeben.

Die klare Lösung wurde auf 38°C erwärmt. Nach drei Stunden bildeten sich die ersten Kristalle. Nach 9 Stunden wurde bis auf 20°C abgekühlt und der voluminöse Niederschlag wurde abgenutscht. Nach Waschen mit Wasser und Trocknen ergaben sich 19,3 g reines L-Leucin mit einem Drehwert $[\alpha]_D^{25}$ = +16,2°. Das Filtrat blieb weitere 24 Stunden bei 20°C stehen, wobei sich erneut Kristalle abschieden. Diese Mischfraktion von 14,6 g mit einem Drehwert $[\alpha]_D^{25}$ = +17,4° wurde abgetrennt und erneut dem Prozeß zugeführt.

Das Filtrat wurde mit konzentrierter Salzsäure bis pH 1 angesäuert und mit 300 ml Ethylacetat in 2 Portionen extrahiert. Die organische Phase wurde unter vermindertem Druck zur Trockne eingedampft und es wurde ein farbloser Rückstand aus 15,3 g N-Acetyl-isoleucin erhalten.

Dieser Rückstand wurde zur Entacetylierung mit 25 ml konzentrierter Salzsäure und 15 ml Wasser 3 Stunden bei 100°C gerührt. Danach wurde auf 10°C gekühlt und das kristallisierte Isoleucin-Hydrochlorid wurde durch Absaugen isoliert. Dieses Hydrochlorid wurde in 200 ml Wasser gelöst und durch eine Säule mit 70 ml eines schwach basischen Anionenaustauschers (Lewatit MP 62) geleitet. Nach Waschen mit Wasser, Einengen des Eluats bei vermindertem Druck bis zu einem dicken Kristallbrei und Absaugen wurden 9,3 g reines L-Isoleucin mit einem Drehwert $[\alpha]_D^{25}$ = +40,3 erhalten.

**Beispiel 2:**

Eine Fraktion neutraler Aminosäuren, die durch chromatographische Trennung eines Proteinhydrolysates und nachfolgende fraktionierte Kristallisation erhalten wurde, hat folgende Zusammensetzung:

Leu 43 %
Ile 56 %
Val 1 %

525 g dieser Mischung wurden in 2 Litern 2N Natronlauge gelöst und auf 0°C gekühlt. Innerhalb einer Stunde wurden 410 ml Acetanhydrid und gleichzeitig 850 ml 5N Natronlauge eingerührt, wobei darauf geachtet wurde, daß der pH-Wert in dem Bereich von pH 10 bis 12 blieb und die Temperatur +5°C nicht überschritt. Nach beendeter Zugabe wurde noch 1 Stunde bei +5°C gerührt.

Mit halbkonzentrierter Schwefelsäure wurde ein pH von 1,6 eingestellt.

Durch Absaugen, Waschen mit Wasser und Trocknen ergaben sich 553 g Feststoff. Dieser wurde aus wässrigem Methanol umkristallisiert, wobei 480 g einer farblosen Mischung aus 50 % N-Acetyl-leucin und 50 % N-Acetyl-isoleucin anfielen.

86,6 g dieser Mischung aus gleichen Teilen N-Acetyl-leucin und N-Acetyl-isoleucin wurden in 400 ml Wasser

suspendiert und mit 50 %iger Natronlauge auf pH 7,0 eingestellt. Es wurden 50 mg $CoCl_2 \times 6H_2O$ und 50 mg handelsübliche Schweinenieren-acylase (1200 U/mg) zugegeben und es wurde mit Wasser bis auf 500 ml aufgefüllt. Die klare Lösung wurde bei 36°C 6 Stunden gerührt. Durch Abnutschen, Waschen und Trocknen wurden 17,9 g reines L-Leucin mit einem Drehwert $[\alpha]_D^{25}$ +15,9° erhalten.

Das Filtrat blieb über Nacht bei 20°C stehen, wobei sich weitere 9,0 g Kristalle abschieden. Der Drehwert betrug $[\alpha]_D^{25}$ = 21,0°.

Diese Mischfraktion wurde abgetrennt und erneut dem Prozeß zugeführt. Das erhaltene Filtrat wurde mit Salzsäure versetzt, bis pH 1 erreicht war,und mit Ethylacetat extrahiert. Die organische Phase wurde eingedampft und der Rückstand (24,6 g) wurde mit 40 ml konzentrierter Salzsäure und 25 ml Wasser 3 Stunden auf 100°C erwärmt. Danach wurde die klare Lösung auf 10°C abgekühlt und das kristallisierte L-Isoleucin-Hydrochlorid abgesaugt.

Durch Lösen des Hydrochlorids in Ethanol und Neutralisieren mit Triethylamin wurden 15,9 g reines L-Isoleucin mit einem Drehwert $[\alpha]^{25}_D$ = 40,2° erhalten.

**Patentansprüche**

1. Verfahren zur Trennung von L-Leucin und L-Isoleucin in Aminosäuregemischen, die 30 bis 65 Gewichtsprozent L-Leucin, 35 bis 70 Gewichtsprozent L-Isoleucin und höchstens 35 Gewichtsprozent an anderen Aminosäuren, jeweils bezogen auf Trockensubstanz, enthalten, dadurch gekennzeichnet, daß man

a) das Aminosäuregemisch in an sich bekannter Weise acetyliert,

b) aus dem rohen Gemisch der Acetylierungsprodukte durch Ansäuern mit einer Mineralsäure ein an N-Acetyl-L-leucin und N-Acetyl-L-isoleucin angereichertes Gemisch an Acetylaminosäuren ausfällt,

c) dieses angereicherte Gemisch in wässriger Lösung mit einer Konzentration zwischen 0,1 und 1,5 Mol/l an N-Acetylaminosäuren ingesgesamt bei einem pH zwischen 6 und 8 und einer Temperatur zwischen 10 und 40°C in Gegenwart eines Effektors einer Verseifung durch eine L-Aminosäureacylase unterwirft, bis 25 bis 95 % des eingesetzten N-Acetyl-L-leucins zur freien Aminosäure verseift sind,

d) aus dem rohen Verseifungsgemisch reines L-Leucin auskristallisiert und abtrennt,

e) aus der nach dem Abtrennen des L-Leucins verbleibenden Mutterlauge N-Acetyl-L-isoleucin isoliert und

f) das N-Acetyl-L-isoleucin in an sich bekannter Weise zur freien Aminosäure verseift.

2. Verfahren nach Anspruch 1, dadurch gekennzeichnet, daß man das in der Verfahrensstufe b) ausgefällte Gemisch an N-Acetylaminosäuren vor Durchführung der Verfahrensstufe c) aus Wasser einem mit Wasser mischbaren aliphatischen Alkohol mit 1 bis 4 Kohlenstoffatomen oder aus einem Gemisch von Wasser und einem solchen Alkohol umkristallisiert.

**Claims**

1. A process for the separation of L-leucine and L-isoleucine in amino acid mixtures containing from 30 to 65% by weight of L-leucine from 35 to 70% by weight of L-isoleucine and at most 35% by weight of other amino acids, based on dry substance in each case, characterised in that,

a) the amino acid mixture is acetylated in known manner

b) a mixture of acetyl amino acids enriched in N-acetyl-L-leucine and N-acetyl-L-isoleucine is precipitated from the crude mixture of acetylation products by acidification with a mineral acid,

c) this enriched mixture is subjected to hydrolysis in an aqueous solution with a concentration of between 0.1 to 1.5 mol/l of N-acetyl amino acids by an L-amino acid acylase overall at a pH of between 6 and 8 and at a temperature of between 10 and 40°C in the presence of an activator, until from 25 to 95% of the N-acetyl-L-leucine used are hydrolysed to free amino acid,

d) pure L-leucine is crystallized from the crude hydrolysis mixture and is separated,

e) N-acetyl-L-isoleucine is isolated from the mother liquor remaining after separation of the L-leucine and

f) the N-acetyl-L-isoleucine is hydrolysed to free amino acid in known manner.

2. A process according to claim 1, characterised in that the mixture of N-acetyl amino acids precipitated in process stage b) is recrystallized before carrying out process stage c) from water, a water-miscible aliphatic alcohol having from 1 to 4 carbon atoms or from a mixture of water and such an alcohol.

**Revendications**

1°) Procédé pour séparer la L-leucine et la L-isoleucine dans des mélanges d'acides aminés qui contiennent 30 à 65 % en poids de L-leucine, 35 à 70 % en poids de L-isoleucine et au maximum 35 % en poids d'autres acides aminés, toujours calculé sur la matière sèche, procédé caractérisé en ce que:

a) on acétyle le mélange d'acides aminés d'une façon connue en soi,

b) on fait précipiter, à partir du mélange brut des produits de l'acétylation et par acidification avec un acide minéral, un mélange d'acides aminés acétylés enrichi en N-acétyl-L-leucine et en N-acétyl-L-isoleucine,

c) on soumet ce mélange enrichi, en solution aqueuse, avec une concentration qui se situe entre 0,1 et 1,5 mole/l en acides aminés acétylés, à un pH entre 6 et 8, et à une température de 10 à 40°C, en présence d'un activateur à une saponification au moyen d'une acylase de L-aminoacides, jusqu'à saponification de 25 à 95 % de la N-acétyl, L-leucine mise en oeuvre en acides aminés libre,

d) on fait cristalliser la L-leucine pure à partir du mélange brut de saponification et la sépare,

e) on isole la N-acétyl-L-isoleucine de la liqueur mère restant après séparation de la L-leucine, et

f) on saponifie la N-acétyl-L-isoleucine d'une façon connue, en acide aminé libre.

2°) Procédé suivant la revendication 1, caractérisé en ce que l'on fait cristalliser, le mélange d'acides aminés N-acétylés précipités dans l'étape b) du procédé, avant d'effectuer l'étape c) du procédé, dans l'eau, un alcool aliphatique a 1 à 4 atomes de carbone, miscible à l'eau, ou un mélange d'eau et d'un alcool de ce type.